# EUROPEAN PATENT SPECIFICATION

(11) **EP 1 833 386 B1**
(45) Date of publication and mention of the grant of the patent: **23.12.2009**
(21) Application number: 05804393.6
(22) Date of filing: 22.11.2005
(51) Int. Cl.: A61B 17/17

(54) **A DRILL GUIDE ASSEMBLY**
BOHRERFÜHRUNGSANORDNUNG
ENSEMBLE DE GUIDAGE DE PERÇAGE

(30) Priority: 26.11.2004 GB 0426000
(43) Date of publication of application: 19.09.2007
(73) Proprietor: Depuy International Limited, Beeston, Leeds LS11 8DT (GB)
(72) Inventor: COLQUHOUN, Callum, Belgrave, VIC 3160 (AU); ROCK, Michael, Leeds LS13 4NF (GB); THOMAS, David, Paul, Wakefield WF2 7EA (GB)
(74) Representative: Belcher, Simon James
(86) International application number: PCT/GB2005/004460
(87) International publication number: WO 2006/056751

(56) References cited:
- DE-A1- 19 945 217
- US-A- 5 207 753
- US-A- 5 306 278
- US-A- 5 423 827
- US-A- 5 634 927
- US-A- 5 810 829
- US-A- 6 066 142
- US-A1- 2003 135 217

## Description

This invention relates to a drill guide assembly for determining the extremities of an area of bone to be removed in order to prepare the bone to receive a component of an orthopaedie joint prosthesis.

An area of bone material may be removed from a bone in preparation for the bone to received a component of an orthopaedic joint prosthesis. The bone material can he cut out using a cutting instrument, such as a bone saw which is inserted through an incision which provides access to the bone. It can be important to remove a portion of bone that requires cuts in at least two planes, for example in order to provide an "L"-shaped cut in the bone. In such circumstances, it is known to use a bone saw to provide the first and second cuts. It can he desirable to minimise the size of the incision, especially in order to reduce the period taken for the patient to recover from the surgery. The size of a cutting instrument can determine the minimum size of the incision.

It can also be important to be able to control the location and dimensions of the cuts accurately. This is because the bone needs to be appropriately shaped and sized in order to receive the prosthesis. Incorrectly located or sized resections can reduce the quality of the fit of component of the joint prosthesis on the bone. If the component of the joint prosthesis is not fitted correctly, then the effectiveness of the prosthetic joint may be reduced. Also, an incorrectly fitted component of prosthesis may cause excessive wear of the prosthetic joint and/or the bone, causing discomfort to the patient, and decreasing the lifetime of the prosthetic joint.

Further, it can be important to minimise the damage to tissue surrounding the bone that is to be cut. Any unwanted damage to surrounding tissue can be caused by the cutting instrument inadvertently contacting the tissue during resection.

US-5423827 discloses a surgical jig for orienting a femoral component of a prosthetic knee prosthesis. The jig comprises a distal plate having two condyle fingers for engaging the posterior side of the femoral condyles and a rotatable drill guide which permits the surgeon to adjust the placements of two drill bores for receiving condyle pins on a femoral component of a knee prosthesis.

US-5634927 discloses an orthopaedic instrumentation assembly for preparing an end of a bone with a drill for receiving a prosthesis. The instrumentation assembly includes a sizing plate which is mountable on the end of a bone and a drill guide having a three bore holes which can be mounted in a single orientation with respect to the sizing plate.

US-6066142 (see preamble of claim 1) discloses a variable position bone drilling guide apparatus for aiding a surgeon in preparing a bone site. The guide includes a drill tower extending from the template which includes two drill passages. The drill passages provide a surgeon with more than one option for sizing a corresponding bone plate.

The present invention provides a drill guide assembly which provides a number of apertures that can each be used to guide a drill in order to remove a portion of bone.

The invention provides an orthopaedic drill guide assembly for guiding a drilling tool into a bone in orthopaedic surgery, which comprises:
a housing which can be fastened to the bone;
a drill guide block which can be fastened to the housing, having an array of at least two closely spaced drill guide bores which define bone tissue which can be drilled by a drill bit extending through the bores towards the surface of the bone, in which the ratio of the distance between the centres of two adjacent ones of the bores to the sum of the radii of those bores is not more than about 1.5, and in which the drill guide block is removably mountable in the housing in first and second orientations so that bone tissue which lies within the array of bores when the drill guide block is mounted in both the first and second orientations, and which is defined by the bores when the drill guide is mounted in the housing in the first orientation, includes bone tissue which is not defined by the bores when the guide block is mounted in the housing in the second orientation.

It is an advantage of the present invention that the area of bone to be removed can be determined before the cutting procedure. Inaccuracies in the cutting caused by human error, are due to difficulties experienced by the surgeon during the cutting procedure, can be reduced by the present invention. Further, as the drill guide block provides at least two drill guide bores that are closely spaced, then it is possible for the surgeon to drill at least two bores that are spaced close to one another. As the surgeon can drill a number of closely spaced bores, the surgeon will then be able to subsequently use another tool, such as a burr tool or saw, in order to remove any excess unwanted bone material that has not been removed by the drilling.

Preferably, the area defined by the array of at least two closely spaced drill guide bores defines not less than 65% of the total area that is desired to be removed from the bone, more preferably not less than 70% of the total area that is desired to be removed from the bone, especially preferably not less than 75% of the total area that is desired to be removed from the bone, for example not less than 80% of the total area that is desired to be removed from the bone.

By removing a significant portion of the area of bone using a drill, and then if necessary removing the rest of the bone with a burr tool, resection of the bone can be achieved without the use of a saw. It has been found that using a drill guide to guide a drill to remove bone, and then subsequently using a burr tool if necessary, can require a smaller incision compared with using a saw. There are particular advantages associated with minimising the size of the incision required, such as reducing patient discomfort and recovery time after the operation.

Preferably, the ratio of the distance between the centres of two adjacent ones of the bores to the sum of the radii of each of those bores is not more than about 1.4, more preferably not more than about 1.3, especially not more than about 1.2, for example not more than 1.1. Such closely spaced bores can provide optimum bone removal.

Preferably, the ratio of the distance between the centres of at least one pair of adjacent bores in the array to the sum of the radii of those bores is not more than about 1, more preferably not more than about 0.9, especially preferably not more than about 0.8, for example not more than about 0.7. Therefore, preferably, there is no wall extending between at least two adjacent bores in the array (i.e. at least a part of the side walls of each of the bores are open). It has been found that such overlapping of adjacent bores can provide for optimum bone removal. Such overlapping of adjacent bores can also be useful when a removable drill guide sleeve (described below) that extends through a bore is used.

Preferably, the sizes of bores in the drill guide block are the same. This can be advantageous as the user will only need to use a single sized drill bit in order to drill the bone in the area defined by the bores. However, the drill guide block can have different size bores. This might be advantageous if corners need to be cut into the bone. The provision of a smaller bore to guide a smaller drill bit at a corner of the area to be removed might be advantageous over the provision of a larger bore to guide a larger drill bit that would be better used in removing larger portions of bone material away from the corner of the area of bone material to be removed.

Preferably, the bores are arranged in an array that extends in two dimensions. Therefore, the area defined by the array can have at least two sides that are greater in size than the diameter of a bore. The area defined by the array can be a polygon, which might be regular or irregular.

The array of bores can include bores in at least first and second lines, with the distances from a bore in the second line to the two closest bores in the first line being approximately equal. This can help ensure that the bone material removed by the drilling using the bores as guides is even across the area defined by the array. Ensuring that the amount of bone material that is removed is even, helps increase the ease by which the subsequent procedure of removing excess bone material using a burr is performed.

When the ratio of the distance between the centres of two adjacent bores to the sum of the radii of those bores is greater than 1, it is preferable that the thickness of the material of the guide block between the adjacent bores is not too small that the strength of the guide block is weakened. The thickness of the material of the guide block between two such adjacent bores must of sufficient thickness so as to be able to provide sufficient support when guiding a drill bit during a drilling procedure. However, it is also preferable that the thickness of the material of the guide block between the two adjacent bores is not too thick, so that the distance between adjacent bores that have been drilled in the bone by a drill bit guided by those bores are so far spaced apart that it is difficult to remove the material between adjacent bores in the bone, for example using a burr tool.

Preferably, when the ratio of the distance between the centres of two adjacent bores to the sum of the radii of those bores is more than 1, the thickness of the material of the guide block between the two adjacent bores is not more than about 6 mm, more preferably not more than about 5 mm, especially preferably not more than about 4 mm, for example, not more than about 3 mm. Preferably, the thickness of the material of the guide block between the two adjacent bores is not less than 2 mm, more preferably, especially not less than 2.5 mm, for example, not less than about 3 mm.

When the bores are arranged in at least first and second lines, the ratio of (i) the distance between the centre of a bore in the second line to the centre of one of the two closest bores in the first line, to (ii) the sum of the radii of those bores, can be less than about 1. When this is the case, preferably, the ratio of (i) to (ii) is not more than about 0.9, especially preferable not more than 0.8, for example not more than about 0.7.

More than one array of closely spaced bores can be provided in the drill guide block. For example, a first array of closely spaced bores can be provided wherein the first array defines a first area of bone material to be removed, and a second array of closely spaced bores can be provided that defines a second area of bone material to be removed. Preferably, adjacent bores can be considered to be part of the same array when the ratio of distance between the centres of the bores to the sum of the radii of the bores is less than about 2, more preferably less than about 1.8, especially less than 1.6, for example less than 1.5.

There are particular advantages associated with being able to remove the drill guide block from the housing. For example, the same housing can be used to receive a number of different drill guide blocks having different characteristics. For example, it might be preferable in some circumstances to use a drill guide block having a smaller number of bores than in other circumstances. Alternatively, different drill guide blocks having a different arrangement of bores might be used to provide different shaped or sized cuts in the bone, or to provide a cut at a different location in the bone. Further still, it might be preferable to have drill guide blocks having bores of different sizes so as to be able to support the drilling of the bone using different sized drill bits. It can also be advantageous to be able to remove the drill guide block from the housing in order to clean the assembly. A yet further advantage of being able to remove the drill guide block is that the drilling procedure can be performed in a number of steps. For example, an area of bone can be removed using a first drill guide block and then a second area of hone can be used by using a second drill guide block that has a second set of bores that define a second area of the bone.

A drill guide block which is removably mountable in the housing the can be mounted in the housing in first and second orientations. For example, after being mounted in a first orientation the drill guide block can be rotated through 180° and then mounted in the housing in the second orientation. Preferably, the bone tissue which is defined by the bores in the first orientation includes bone tissue which is his not defined by the bores when the guide block is mounted in the second orientation. Therefore, the same drill guide block can be used to remove bone material by drilling the bone in two successive steps, wherein the second step involves the removal of bone material that has not been removed in the first step.

Preferably, the total area defined by the array of at least two closely spaced drill guide bores in the drill guide block's first and second orientations is not less than 80% of the total area that is desired to be removed from the bone, more preferably not less than 85% of the total area that is desired to be removed from the bone, especially preferably not less than 90%.

The drill guide block can have at least one opening extending through it those transverse size is smaller than that of the drill guide bores, and the assembly can includes a fixing pin which can be fitted into the opening and extended into the bone, to fasten the guide block to the bone. This can be advantageous when the guide block is removable from the housing as the fixing pin will ensure that the drill guide block does not become loose or be displaced from its position in the housing during the drilling procedure. Further, the bone that is removed due to the insertion of the fixing pin can form part of the total area of the bone that is to be removed. Therefore, when the area of bone to be removed includes a corner, it can be preferable to arrange the drill guide block so that the opening is positioned at the location where the corner of the cut from the bone will be.

The size of each bore should be sufficient for a drill bit to be a sliding fit within it, with minimum clearance which would allow play to reduce the accuracy of the location of the drill bit that is used to drill into the bone. Preferably, the internal width of the bores is not less than 2 mm, more preferably, not less than 3 mm, especially preferably, not less than 4 mm, for example 5 mm. Preferably, the internal width of the bore is not more than 10 mm, more preferably not more than 8 mm, especially preferably not more than 6 mm, for example not more than 3 mm.

The drill guide assembly can comprise a removable drill guide sleeve that extends through one of the bores in the drill guide block to protect tissue surrounding the bone from a drill bit extending through the bore. The drill guide sleeve will protect the surrounding tissue from the rotating drill so as to prevent damage caused to the tissue by the drill bit. Preferably, the drill guide sleeve is of a size that fits snugly within the bores so that the friction caused between the drill guide sleeve and the bore prevents the drill guide sleeve rotating within the bore during the drilling procedure. Preferably, the internal width of the sleeve is not less than 2 mm, more preferably, not less than 3 mm, especially preferably, not less than 4 mm, for example 5 mm. Preferably, the internal width of the sleeve is not more than 10 mm, more preferably not more than 8 mm, especially preferably not more than 6 mm, for example not more than 3 mm. Preferably, when the drill guide assembly comprises a removable drill guide sleeve, the diameter of the bores are larger than a drill guide assembly which does not include a removable drill guide sleeve so as to accommodate the drill guide sleeve without reducing the maximum diameter drill bit that can extend through the drill guide assembly.

The guide sleeve is mounted within a housing which can be fastened either directly or indirectly to the bone. Preferably the housing is fastened indirectly to the bone by a jig instrument which can be fastened to a bone. Preferably, once fastened to the bone the jig can be adjusted so that the position of the mounting of the guide sleeve relative to the bone can be adjusted. Preferably, the position of the mounting can be adjusted in the medial/lateral, proximal/distal and posterior/anterior dimensions relative to the bone. Preferably, the rotational position of the mounting can be adjusted about x, y and z axes (i.e. three axes, each perpendicular to one another) relative to the bone. Preferably, the position of the mounting can be adjusted through the use of adjustment screws on the housing and/or the jig. However, any means for adjusting the position of the mounting can be used. Jig instruments can receive surgical instruments, cutting blocks or tools which can be fastened to the bone and subsequently adjusted so that the position of the tool or instrument can be accurately located relative to the bone are well known. Such jig instruments of this general kind are known, including those available from DePuy Products, Inc under the name CAS Preservation Tibial Jig (which is used as part of DePuy's MI TKR System surgical technique), the ankle clamp based jig which forms part of the knee replacement instrument set under the trade mark Specialist 2.

When the drill guide block is removable from the housing, the mounting in which the drill guide block is fastened can also be used to pivotally mount a burr guide sleeve for guiding a burr tool in a plane to resect the bone. Preferably, the assembly further includes a guide sleeve which can be pivotally mounted in the housing, the guide sleeve having a bore extending along its length in which a burr tool can be received such that the cutting portion of the burr tool extends from the sleeve towards the bone which is to be cut, the sleeve being mounted in the housing for pivotal movement around an axis so that the cutting portion of the burr tool is restricted to movement in a plane which is perpendicular to that axis; and a burr tool which is a sliding fit in the guide sleeve.

The assembly will generally be made from metallic based materials which are conventionally used in the manufacture of surgical instruments. Certain stainless steels can be particularly preferred. However, it will be understood that at least one part of the assembly, for instance the drill guide block, can be made from polymeric materials. Using polymeric materials can reduce the cost of manufacture of the assembly, especially because an assembly made from polymeric materials can easily be manufactured using a moulding process. When the drill guide block is made from a polymeric material, it can be preferred that a removable drill guide sleeve that extends through a bore in the drill guide block is present, in order to protect the drill guide block from the drill bit extending through the bore. Suitable polymeric materials include certain polycarbonates, polyester, polyamides, poly-ether-ether ketones (PEEKs), and polyaryl-ether ketones (PAEKs). Polymeric materials can be reinforced with particulate material, especially fibrous materials, to provide appropriate wear characteristics.

The embodiments of the present invention are described by way of example only with reference to the accompanying drawings in which:
Figure 1 is a perspective view of an assembly according to the present invention, mounted on the proximal tibia.
Figure 2a is a perspective view of the drill guide block of the assembly show in Figure 1, according to a first embodiment.
Figure 2b is a front view of the drill guide block shown in Figure 2a.
Figure 2c is a top view of the drill guide block shown in Figure 2a.
Figure 2d is a side view of the drill guide block shown in Figure 2a, wherein the phantom lines illustrate the area of bone that will be removed through use of the drill guide block in its first and second orientations.
Figure 3a shows a perspective view of the drill guide block and guide sleeve of the assembly shown in Figure 1.
Figure 3b is a side view of the drill guide block and the guide sleeve shown in Figure 3a.
Figure 3c is a top view of the drill guide block and guide sleeve shown in Figure 3a.
Figure 3d is a end view of the drill guide block and guide sleeve shown in Figure 3a.
Figure 4 is a side view of the drill guide block of ths assembly shown in Figure 1, according to a second embodiment.
Figure 5 is a perspective view of an assembly according to a second embodiment of the invention, mounted on the femur.
Figure 6 is a perspective view of the assembly according to the second embodiment shown in Figure 5 in isolation.
Figure 7a is a side view of the assembly according to the second embodiment shown in Figure 5.
Figure 7b is a top view of the assembly according to the second embodiment shown in Figure 5.
Figure 8 is a perspective view of the housing according to the second embodiment of the invention in which a burr guide sleeve is mounted.

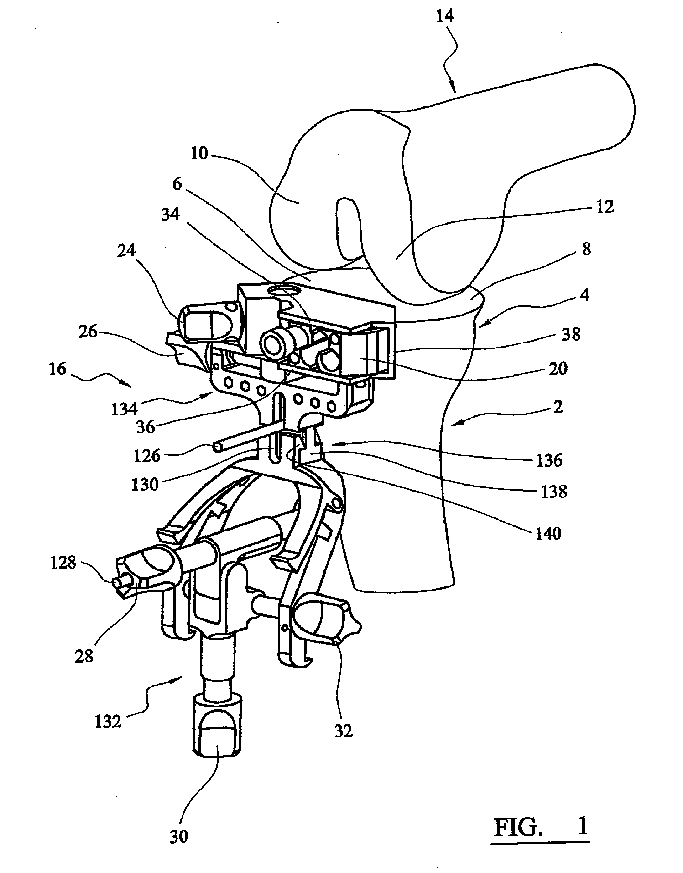

Referring to the drawings, Figure 1 shows the proximal portion of a tibia 2, including the tibial plateau 4 of the tibia which has medial 8 and lateral 6 condials for receiving the medial 12 and lateral 10 condyles of the distal part of a femur 14. The tibia 2 and femur 14 are shown in isolation to show how the present invention can be put into effect to prepare the tibia for implantation of a component of an orthopaedic joint prosthesis. The prosthesis can be used to provide a hard-wearing bearing surface on the proximal tibia 2, which can articulate with an appropriate prosthesis component which is implanted on the distal end of the femur 14. Although the invention is described in relation to preparing the proximal tibia 2, the present invention can be used to prepare any part of any bone, for example, the distal or proximal end of a femur, or the proximal or distal end of a humerus.

The preparation of the proximal tibia to receive a component of an orthopaedic joint prosthesis involves the removal of an area of bone on the tibia proximal tibia, to receive a part of the component having a shape corresponding to that of the area of bone removed.

Figure 1 shows a drill guide assembly 16 according to the present invention fastened to the tibia 2. The drill guide assembly generally comprises a housing 134 which can be fastened indirectly to the tibia 2 by a jig 132, and a drill guide block 20 which is mounted in the housing. The housing 134 provides a mounting 22 in which the drill guide block 20 can be removably mounted.

The jig 132 has first 28, second 30 and third 32 adjustment screws which can be rotated to adjust the tibial slope, distal/proximal and varus/valgus positioning of the mounting 22 relative to the tibia, respectively. The housing 134 comprises a fourth 24 and fifth 26 adjustment screws which can be rotated to adjust and lock the internal/external rotational, and to adjust the medial/lateral position of the mounting 22 relative to the tibia, respectively.

The jig 132 can be fastened to the bone through the use of first 126 and second 128 fixing pins. The first fixing pin 126 extends through a channel 130 defined by the jig 132 and the housing 134. The channel 130 is elongated so as to allow the adjustment of the proximal/distal position of the housing 134 relative to the tibia once the first fixing pin 126 has been fastened to the tibia. The first adjustment screw 28 is cannulated, and the second fixing pin 128 extends through first adjustment screw.

The jig 132 and housing 134 each define a portion of the channel 130 through which the first fixing pin 126 extends (i.e. the jig 132 and housing 134 each provide a U-shaped recess at their interface). The jig 132 and housing 134 can be releasably fastened to one another at their interfaces by a push release latch mechanism 136. However, it will be understood that other mechanisms other than a latch can be used to releasably fasten the housing 134 to the jig 132. The latch mechanism comprises a flap 138 provided on the housing 134 and a hook 140 provided on the jig 132. However, it will be understood that the flap can be provided on the jig 132 and the hook can be provided on the housing 134. When in the engaged position, a flat side of the flap 138 is held against a flat side of the hook 140 so as to prevent the jig 132 and housing 134 moving away from each other. The housing 134 can be released from the jig 132 by pulling the flap 138 away from the hook 140. It will be understood that the housing 134 and jig 132 need not be separate components (i.e. they can be one piece).

The jig 134 is a standard jig for receiving a surgical instrument, cutting block or tool so that its position can be accurately located and fixed, either directly or relative to the bone. Such jig instruments of this general kind are known, including those available from DePuy Products, Inc under the name CAS Preservation Tibial Jig (which is used as part of DePuy's MI TKR System surgical technique).

The mounting 22 comprises two opposing parallel walls 34, 36 and two end walls 38 (second end wall not shown) extending between the two parallel walls at either end of the parallel walls. The end walls 38 are angled so that when the assembly is in use the distance between the end walls decreases as you travel towards the tibia 2. Therefore, the cross-sectional shape of the mounting 32 is generally the shape of an isosceles trapezoid. A mounting having such configurations can be beneficial when the mounting is also to be used to receive a burr guide sleeve as described in more detail below. It will be appreciated that the mounting can have a cross-sectional shape other than that of an isosceles trapezoid. For example, the cross-sectional shape of the mounting 22 can be square or rectangular.

The parallel walls 34, 36 are separated by a distance that is slightly larger than the height of the drill guide block 20 and the end walls 38 are separated by a distance that is slightly larger than the width of the drill guide block. The distances are large enough such that the drill guide block 20 is capably of easily sliding between the walls into the mounting, but small enough such that any translational or rotational movement of the drill guide block 20 within the mounting 22 is prevented once the drill guide block has been slid into the mounting.

The mounting 22 of the housing 134 can contain formations (not shown) which engage with corresponding formations in a drill guide block 20 so as to ensure proper seating of the drill guide block within the mounting 22. The formation of the mounting 22 comprises two cylindrical projections (not shown) which are located on the inside of the opposing parallel walls 34, 36 of the mounting 22 so that they are co-axial. It will be understood that other formations can be used and that it is not essential that the formations are cylindrical projections. For example, the formations can be cuboidal projections. Further, it will be understood that it is not essential to provide any formations. For example, the proper seating of the drill guide block 20 within the mounting 22 can be ensured by the engagement of the walls of the drill guide block 20 with the walls of the mounting 22. However, it can be beneficial to provide formations, in particular cylindrical projections, to provide a fulcrum about which a separate burr guide sleeve can pivot when the mounting 22 can also receive a burr guide sleeve as described in more detail below.

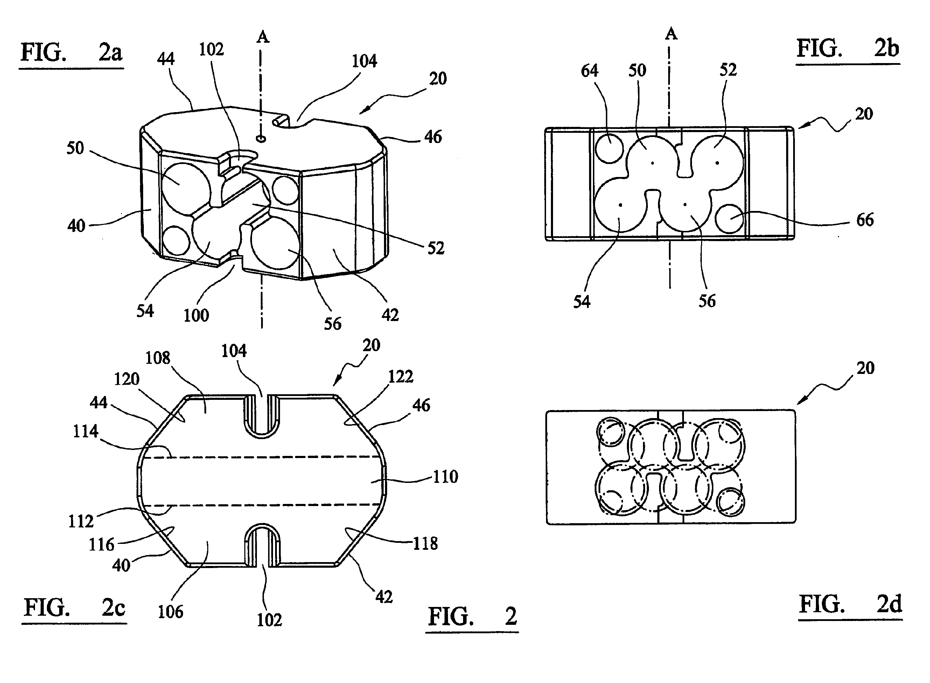

Figure 2 shows the drill guide block 20 in more detail. As best shown in Figures 2a and 2c, the cross-sectional shape taken perpendicular to an axis A is generally that of identical first and second isosceles trapezoids 106, 108 that are each joined at their bases 112, 114 to the sides of a rectangular section 110 having a length equal to the bases of the isosceles trapezoids. The ends 116, 118 of the first isosceles trapezoid 106 that extend between the parallel sides of the first isosceles trapezoids define first 40 and second 42 angled end walls of the drill guide block 20. Further, the ends 120, 122 of the first isosceles trapezoids that extend between the parallel sides of the second isosceles trapezoid 108 define third 44 and fourth 46 angled end walls of the drill guide block 20. This configuration allows the drill guide block 20 to be mounted in the mounting 22 in two orientations. When mounted in the first orientation, the first and second angled end walls 40, 42 of the drill guide block 20 abut the end walls 38 of the mounting 22. When mounted in the second orientation the third and fourth angled end walls 44, 46 of the drill guide block 20 abut the end walls 38 of the mounting 22. It will be appreciated that the drill guide block 20 can have a different cross-sectional shape. For example, the cross-sectional shape of the drill guide block can be square or rectangular. In such configurations, the mounting 22 will be shaped and sized appropriately to receive the square or rectangular drill guide block.

The drill guide block 20 has first 100, second 102, third 104 and fourth (not shown) recesses for engagement with the formations (not shown) on the parallel walls 34, 36 of the mounting 22. When the drill guide block is mounted in its first orientation, the first 100 and second 102 recesses engage the formations on the parallel walls 34, 36 of the mounting 22. When the drill guide block is mounted in its second orientation, the third 104 and fourth (not shown) recesses engage the formations on the parallel walls 34, 36 of the mounting 22.

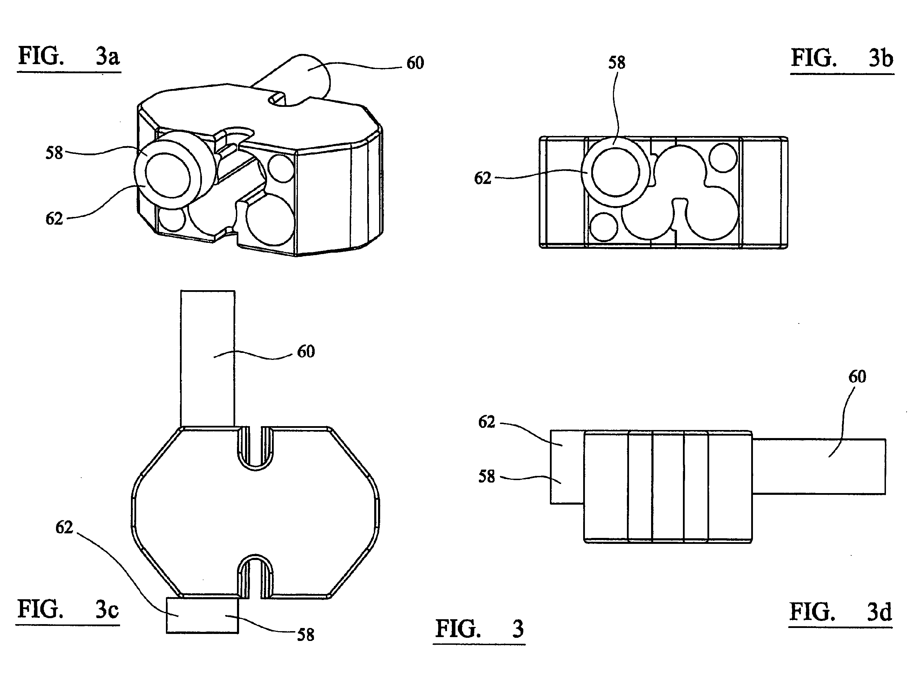

The drill guide block 20 further comprises an array of four closely spaced drill guide bores 50, 52, 54, 56 which extend through the drill guide block 20. The axes of the four closely spaced drill guide bores 50, 52, 54, 56 are perpendicular to the axis A. The diameters of the bores 50, 52, 54, 56 are slightly bigger than the size of a drill sleeve (designated by reference numeral 58 shown in Figure 3) which can extend through the bores. The bores are arranged in first and second lines, with the first line comprising first 50 and second 52 bores and the second line comprising third 54 and fourth 56 bores. A line extending between the centre lines of the bores of the first line extends perpendicular to the axis A. A line extending between the centre lines of the bores of the second line also extends perpendicular to the axis A. The centre line of a bore is the line that extends along longitudinal axis of the bore. The distance from the centre lines of the first bore 50 and the centre lines of the third and fourth bores 54, 56 are equal. Further, the distance from the centre line of the fourth bore 56 to the centre lines of the first and second bores 50, 52 are equal.

The distance between the centre lines of the first bore 50 and the third bore 54 is less than the sum of the radii of the bores. In particular, the ratio of the distance between the centre lines of the first and third bores 50, 54 to the sum of the radii of the bores is approximately 0.8. Therefore, there is no wall extending between the first and third bores 50, 54 as shown in Figures 2a and 2b (i.e. at least a part of the side walls of each of the bores are open). Further, the ratio of the distance between the centre lines of the first and fourth bores 50, 56 to the sum of the radii of the bores is also approximately 0.8. Further still, the ratio of the distance between the centre lines of the second and fourth bores 52, 56 is also approximately 0.8.

The ratio of the distance between the first and second bores and the sum of the radii of the bores is approximately 1.2. Further, the ratio of the distance between the third and fourth bores 54, 66 to the sum of the radii of the bores is also approximately 1.2. Therefore, a portion of wall exists between the first and second bores 50, 52 and also between the third and fourth bores 54, 56 as shown in Figures 2a and 2b.

When the drill guide block 20 is mounted in the mounting 22 and housing 134 is fastened to the tibia 2 by the jig 136, the array of bores 50, 52, 54, 56 defines an area of the tibia 2. A drill bit can be used to remove the area of bone defined by the array of bores 50, 52, 54, 56 by passing the drill bit through each of the bores in successive drilling steps. Further, as discussed above, the drill guide block 20 can be removed from the mounting 22 and remounted in the mounting in a second orientation. The drill guide block 20 is rotated by 180° about axis A when mounted in its second orientation relative to its first orientation. Accordingly, the total area of the tibia hear that can be defined by the array of bores 50, 52, 54, 56 comprises the area defined by the array when the drill guide block 20 is in its first orientation and also when the drill guide block is in its second orientation. Figure 2d illustrates the side view of the drill guide block 20 when in its first orientation. Further, the phantom lines show the total area of bone that will be removed through the use of a drill bit that has been guided by each of the bores of the drill guide block 20 in both its first and second orientation.

The drill guide block 20 also comprises fifth and sixth bores 64, 66. The diameters of the fifth and sixth bores 64, 66 are smaller than that of the first, second, third and fourth bores 50, 52, 54, 56. As shown in this embodiment, the fifth and sixth bores 64, 66 form part of the array of bores which defines the area of bone to be removed. As shown in Figure 2b, the fifth and sixth bores 64, 66 are located at diametrically opposite ends of the array and can be used to guide a drill bit to remove bone material from the corners of the area of bone to be removed. The fifth and sixth bores 64, 66 can also be used to receive fixing pins (not shown). The fixing pins can be fitted through the fifth and sixth bores 64, 66 and can extend into the tibia 2 so as to fasten the drill guide block 20 to the tibia.

Figure 3 shows different views of the drill guide block 20 wherein a drill guide sleeve 58 has been passed through the first bore 50. The drill guide sleeve 58 comprises a sleeve portion 60 and a head portion 62. The diameter of the sleeve portion is slightly smaller than that of the diameter of the bores 50, 52, 54, 56 so that the sleeve can slide within the bores. The diameter of the head portion 62 of the drill guide sleeve 58 is slightly larger than that of the bores 50, 52, 54, 56 so that the head portion is prevented from sliding into the bores and thereby limits the amount by which the drill guide sleeve 58 can pass through the bores. The sleeve portion 60 is greater than the depth of the drill guide block 20 taken parallel to the axes of the bores. The internal diameter of the drill guide sleeve 58 is slightly larger than that of a drill bit that is to be passed through the sleeve in order to remove bone material.

Although the embodiment of the assembly includes a drill guide sleeve 58, it will be understood that it is not essential that a drill guide sleeve be used or included. For example, the drill bit can extend and be guided by the bores 50, 52, 54, 56 without the use of a drill guide sleeve.

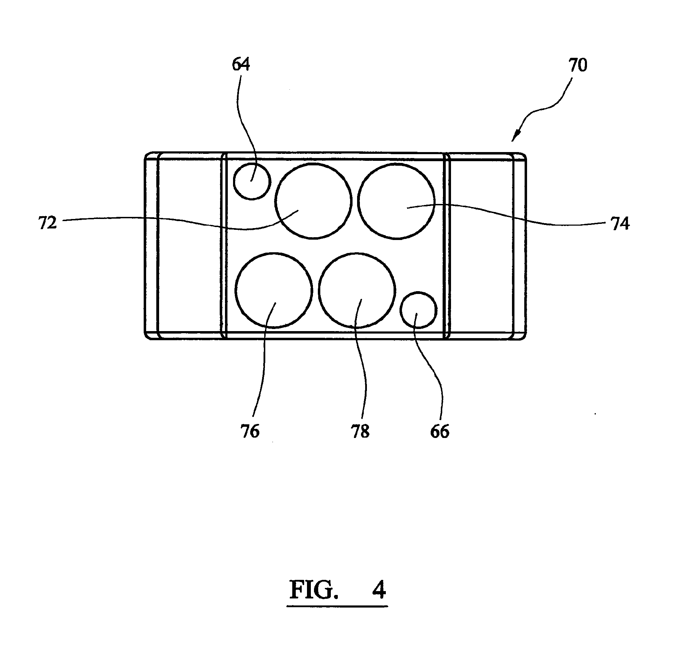

Figure 4 shows a side view of a second embodiment of the drill guide block 70 according to the present invention. The second embodiment of the drill guide block 70 is similar to that shown in Figures 1, 2 and 3 and like parts share like reference numerals. However, the first 72, second 74, third 76 and fourth 78 bores are spaced so that a portion of wall extends between each of the bores. The ratio of the distance between the centre lines of the first and third bores 72, 76 to the sum of the radii of the bores is approximately 1.2. Further, the ratio of the distance between the centre lines of the first and fourth bores 72, 78 to the sum of the radii of the bores is also approximately 1.2. Further still, the ratio of the distance between the centre lines of the second and fourth bores 74, 78 to the sum of the radii of the bores is also approximately 1.2. Furthermore, the ratio of the distance between the centre lines of the first and second bores 72, 74 to the sum of the radii of the bores is approximately 1.2. Finally, the ratio of the distance between the centre lines of the third and fourth bores 76, 78 to the sum of the radii of the bores is approximately 1.2.

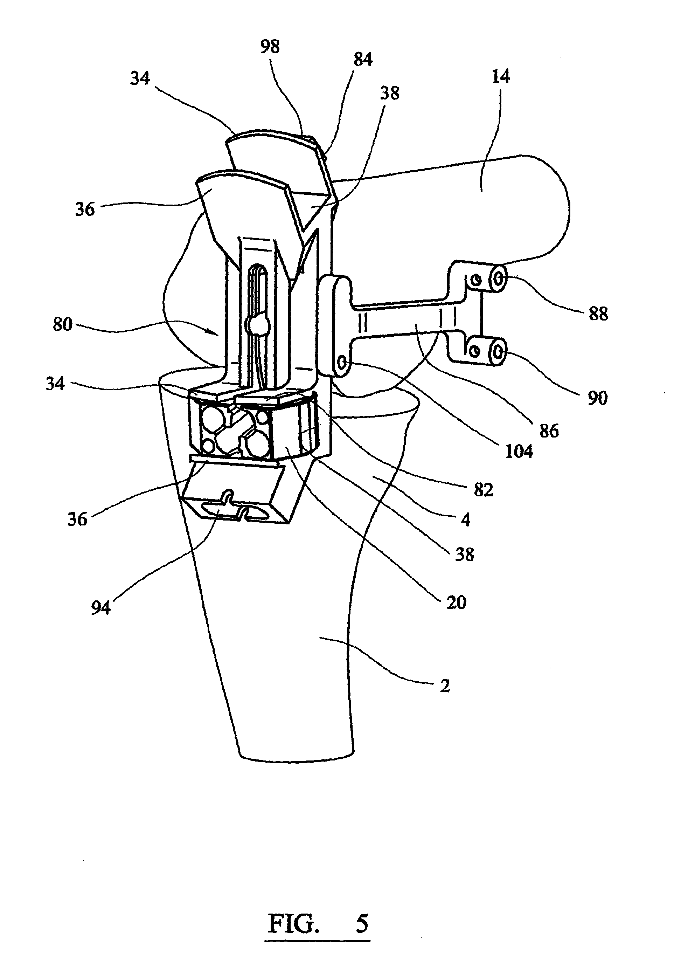

Figure 5 shows a second embodiment of the housing 134 according to the present invention. The second embodiment of the housing 80 provides a first mounting 82 and a second mounting 84 in which the drill guide block 20 can be mounted. The first and second mountings 82, 84 are similar to the mounting 22 of the housing 134, and like parts share like reference numerals. The first and second mountings 82, 84 comprise opposing parallel walls 34, 36 and first 38 and second (not shown) end walls extending between the parallel walls. The end walls 38 are angled so that when the assembly is in use the distance between the end walls decreases towards the bone. Also, there are formations (not shown) in the form of cylindrical projections on the inside of the parallel walls with which the recesses 100, 102, 104 in the drill guide block 20 can engage. The first and second mountings 82, 84 can be used to mount a drill guide block 20 to guide a drill bit to remove bone material from a bone, such as the femur 14.

The housing 80 can be fastened to the femur 14 by means of a bracket 86. The bracket 86 is fastened at a first end to the housing via means of a fixing pin (not shown) that extends through a first bore 124 in the bracket into the housing, and can be fastened to the femur at a second end by means of fixing pins (not shown) that extend through second and third bores 88, 90 in the bracket. In order to further secure the housing 80 to the femur 14, the housing 80 also provides a fourth bore 92 that extends through a block 98 located on the second mounting 84. The fourth bore 92 can receive a fixing pin (not shown) that can inserted into the femur 14 so as to fasten the housing 80 to the femur.

The housing 80 also provides a slot 94 through which a burr tool or other surgical instrument can be inserted for providing a further resection or operation on the femur 14, for example, for performing a resection on the femur 14.

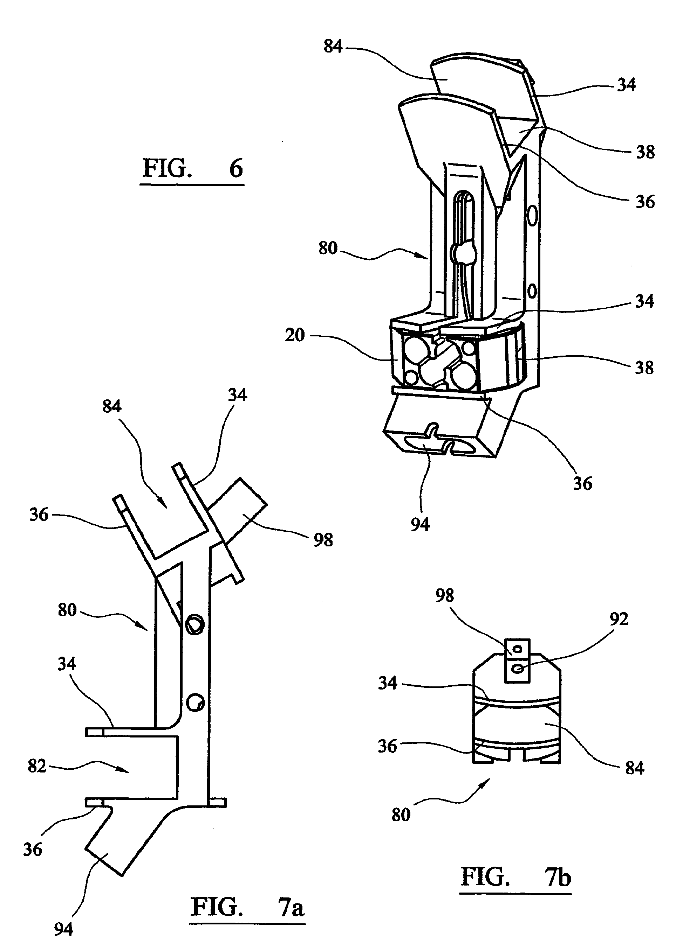

Figure 6 shows a perspective view of the housing 80 in isolation. Figure 7a shows the side view of the housing 80 without the drill guide block 20 located within it.

Typically, only the first mounting 82 will be used to mount a drill guide block 20 to guide a drill bit to remove bone material from, for example, the femur 14. However, the use of a housing 80 having two mountings 82, 84 can be useful when using a burr guide sleeve to guide a burr tool in two planes to perform a two resections on the femur 14 in successive steps. This is because the housing 80 can be used to provide suitable mountings for the burr guide sleeve 38 to guide a burr tool to perform such resections without the need to accurately locate and fasten the housing in two locations to perform the two resections. Instead, the housing 80 with two mountings 82, 84 can be located and fastened to the bone just once to provide two suitable mountings.

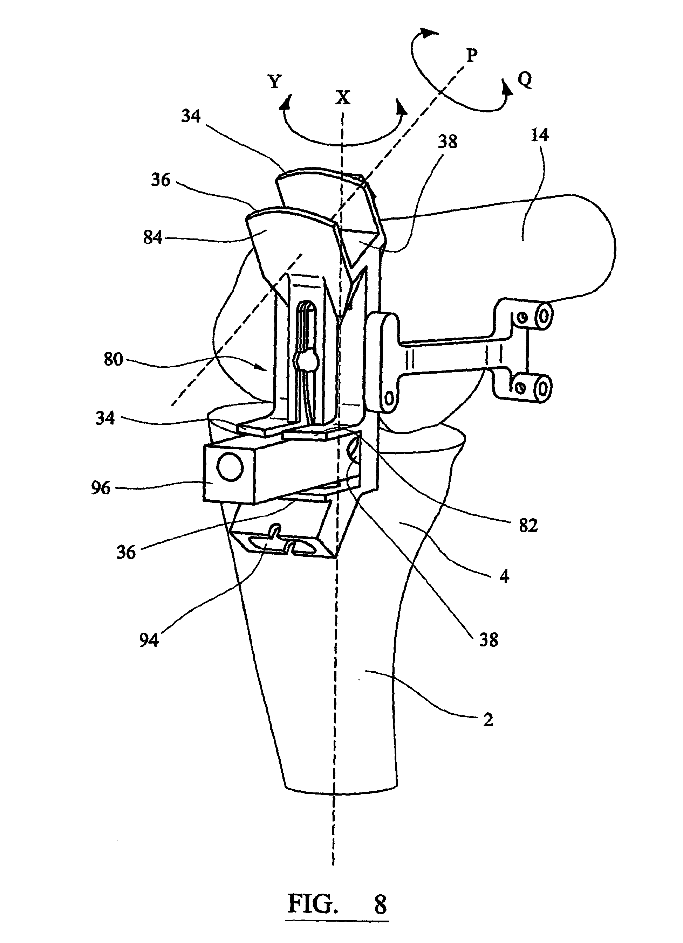

Figure 8 shows an embodiment of the housing 80 in which a burr guide sleeve 96 is mounted in the first mounting 82. The burr guide sleeve 96 is pivotedly mounted in the mounting 82 by means of formations (not shown) in the form of semi-circular recesses located in the end of the burr guide sleeve 96 which engage with cylindrical projections (not shown) located on the inside of the opposing parallel walls 34, 36 of the first mounting. Therefore, the cylindrical projections define an axis X about which the burr guide sleeve 96 can pivot in the direction shown by arrow Y. The end walls 38 of the mounting 82 act as stops which restrict the amount by which the burr guide sleeve 96 can pivot about the axis A in the direction shown by arrow B. The burr guide sleeve 96 can be removed from the first mounting 82 and mounted in the second mounting 84 of the housing 80. Similarly, the second mounting 84 comprises cylindrical projections (not shown) located on the inside of the opposing parallel walls 34, 36 with which the semi-circular shown) located on the inside of the opposing parallel walls 34, 36 with which the semi-circular recesses located in the end of the burr guide sleeve 96 can engage. Therefore, the cylindrical projections define an axis P about which the burr guide sleeve 96 can pivot in the direction shown by arrow Q.

The burr guide sleeve can be used for guiding a burr tool during a procedure in which the burr tool is used to cut the bone. For example, when mounted in the first mounting 82, the burr guide sleeve 96 can be used to guide a burr tool to perform a first resection of the femur 14 and when mounted in the second mounting 84 can be used to guide a burr tool to perform a second resection of the femur. Further, the burr guide sleeve 96 can be mounted in the first mounting 82 and used to guide a burr tool to resect a portion of the femur 14, subsequent to the removal of an area of bone material from the femur through the use of a drill bit guided by the drill guide block 20 mounted in the first mounting 82.

Although the second embodiment of the housing 80 is described in relation to removing bone material from/performing resections on a femur, it will be understood that this housing can also be fastened to and used for removing bone material from a tibia, or other bones, for example the humerus.

## Claims

1. An orthopaedic drill guide assembly for guiding a drilling tool into a bone in orthopaedic surgery, which comprises:
a housing (134) which can be fastened to the bone;
a drill guide block (20) which can be fastened to the housing (134), having an array of at least two closely spaced drill guide bores (50, 52, 54, 56) which define bone tissue which can be drilled by a drill bit extending through the bores towards the surface of the bone, in which the ratio of the distance between the centres of two adjacent ones of the bores to the sum of the radii of those bores is not more than about 1.5,
**characterised in that** the drill guide block is removably mountable in the housing in first and second orientations so that bone tissue which lies within the array of bores when the drill guide block is mounted in both the first and second orientations, and which is defined by the bores when the drill guide is mounted in the housing in the first orientation, includes bone tissue which is not defined by the bores when the guide block is mounted in the housing in the second orientation.

2. An assembly as claimed in claim 1, in which the bores (50, 52, 54, 56) are arranged in a regular pattern extending in two dimensions.

3. An assembly as claimed in claim 1, in which the bores (50, 52, 54, 56) are arranged in at least first and second lines, with the distances from a bore in the second line to the two closest bores in the first line being approximately equal.

4. An assembly as claimed in claim 1, in which the drill guide block is rotated through 180° between the first and second orientations.

5. An assembly as claimed in claim 1, in which the ratio of the distance between the centres of at least one pair of adjacent bores in the array to the sum of the radii of those bores is not more than about 1.

6. An assembly as claimed in claim 1, in which the thickness of the material of the guide block (20) between two adjacent bores is not more than about 3 mm.

7. An assembly as claimed in claim 1, in which the guide block (20) has at least one opening (64, 66) extending through it whose transverse size is smaller than that of the drill guide bores (50, 52, 54, 56), and in which the assembly includes a fixing pin which can be fitted into the opening and extend into the bone, to fasten the guide block to the bone.

8. An assembly as claimed in claim 1, which includes a removable drill guide sleeve (58) that extends through one of the bores (50, 52, 54, 56) in the drill guide block (20) to protect tissue surrounding the bone by a drill extending through the bore.

9. An assembly as claimed in claim 1, which includes:
a guide sleeve (96) which can be pivotally mounted in the housing (80), the guide sleeve having a bore extending along its length in which a burr tool can be received such that the cutting portion of the burr tool extends from the sleeve towards the bone which is to be cut, the sleeve being mounted in the housing for pivotal movement around an axis (X) so that the cutting portion of the burr tool is restricted to movement in a plane which is perpendicular to that axis; and
a burr tool which is a sliding fit in the guide sleeve.

## Patentansprüche

1. Orthopädische Bohrführungsanordnung zum Führen eines Bohrwerkzeuges in einen Knochen bei der orthopädischen Chirurgie, die aufweist:
ein Gehäuse (134), das an dem Knochen festgelegt werden kann;
einen Bohrführungsblock (20), der an dem Gehäuse (134) festgelegt werden kann, mit einer Anordnung aus wenigstens zwei nahe beieinander liegenden Bohrführungsbohrungen (50, 52, 54, 56), die Bohrgewebe definieren, das von einer Bohrspitze gebohrt werden kann, welche sich durch die Bohrungen auf die Oberfläche des Knochens zu erstreckt, wobei das Verhältnis der Abstände zwischen den Mittelpunkten zweier Benachbarten der Bohrungen zur Summe der Radien dieser Bohrungen nicht mehr als 1.5 beträgt,
**dadurch gekennzeichnet, dass** der Bohrführungsblock entfernbar in dem Gehäuse in einer ersten und einer zweiten Ausrichtung anbringbar ist, so dass Knochengewebe, das innerhalb der Anordnung der Bohrungen liegt, sowohl wenn der Bohrführungsblock in der ersten als auch wenn er in der zweiten Ausrichtung angebracht ist, und das durch die Bohrungen definiert ist, wenn die Bohrführung in dem Gehäuse in der ersten Ausrichtung angebracht ist, Knochengewebe umfasst, das nicht durch die Bohrungen definiert ist, wenn der Führungsblock in dem Gehäuse in der zweiten Ausrichtung angebracht ist.

2. Anordnung nach Anspruch 1, bei der die Bohrungen (50, 52, 54, 56) in einem regelmäßigen Muster angeordnet sind, das sich in zwei Dimensionen erstreckt.

3. Anordnung nach Anspruch 1, bei der die Bohrungen (50, 52, 54, 56) in wenigstens einer ersten und einer zweiten Reihe angeordnet sind, wobei die Abstände von einer Bohrung in der zweiten Reihe zu den zwei nächsten Bohrungen in der ersten Reihe ungefähr gleich ist.

4. Anordnung nach Anspruch 1, bei der der Bohrführungsblock zwischen der ersten und der zweiten Ausrichtung um 180° gedreht wird.

5. Anordnung nach Anspruch 1, bei der das Verhältnis des Abstandes zwischen den Mittelpunkten wenigstens eines Paares benachbarter Bohrungen in der Anordnung zu der Summe der Radien dieser Bohrungen nicht mehr als ungefähr 1 ist.

6. Anordnung nach Anspruch 1, bei der die Dicke des Materials des Führungsblockes (20) zwischen zwei benachbarten Bohrungen nicht mehr als ungefähr 3 mm ist.

7. Anordnung nach Anspruch 1, bei der der Führungsblock (20) wenigstens eine Öffnung (64, 66) hat, die sich durch ihn hindurch erstreckt, deren Querabmessung kleiner ist als die der Bohrführungsbohrungen (50, 52, 54, 56), und wobei die Anordnung einen Fixierstift umfasst, der in die Öffnung gesetzt werden kann und sich in den Knochen erstreckt, um den Führungsblock an dem Knochen zu befestigen.

8. Anordnung nach Anspruch 1, die eine entfernbare Bohrführungshülse (58) umfasst, die sich durch eine der Bohrungen (50, 52, 54, 56) in dem Bohrführungsblock (20) erstreckt, um Gewebe, das den Knochen umgibt, vor einem Bohrer zu schützen, der sich durch die Bohrung erstreckt.

9. Anordnung nach Anspruch 1, die umfasst:
eine Führungshülse (96), die schwenkbar in dem Gehäuse (80) angeordnet werden kann, wobei die Führungshülse eine Bohrung hat, die sich über ihre Länge erstreckt, in der ein Entgratungswerkzeug aufgenommen werden kann, so dass sich der Schneidbereich des Entgratungswerkzeugs von der Hülse auf den Knochen, der geschnitten werden soll, zu erstreckt, wobei die Hülse in dem Gehäuse für die Schwenkbewegung um eine Achse (X) angebracht ist, so dass der Schneidbereich des Entgratungswerkzeugs auf die Bewegung in einer Ebene, die senkrecht zu dieser Achse ist, eingeschränkt ist; und
ein Entgratungswerkzeug, das verschieblich in der Führungshülse sitzt.

## Revendications

1. Ensemble de guidage de foret orthopédique destiné à guider un outil de forage dans un os en chirurgie orthopédique, qui comprend :
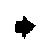 un logement (134) qui peut être fixé sur l'os ;
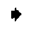 un bloc de guidage de foret (20) qui peut être fixé sur le logement (134), qui présente un réseau de deux alésages de guidage de foret au moins très proches les uns des autres (50, 52, 54, 56) qui définissent un tissu osseux qui peut être foré à l'aide d'un outil de forage qui s'étend à travers les alésages vers la surface de l'os, dans lequel le rapport de la distance qui sépare les centres de deux alésages adjacents sur la somme des rayons de ces alésages, n'est pas supérieur à 1,5 environ ;
**caractérisé en ce que** le bloc de guidage de foret peut être monté de manière amovible dans le logement dans des première et seconde orientations de telle sorte que le tissu osseux qui se trouve en dessous du réseau d'alésages lorsque le bloc de guidage de foret est monté dans les première et seconde orientations, et qui est défini par les alésages lorsque le bloc de guidage de foret est monté dans le logement dans la première orientation, comprenne le tissu osseux qui n'est pas défini par les alésages lorsque le bloc de guidage de foret est monté dans le logement dans la seconde orientation.

2. Ensemble selon la revendication 1, dans lequel les alésages (50, 52, 54, 56) sont disposés en un motif régulier qui s'étend dans deux dimensions.

3. Ensemble selon la revendication 1, dans lequel les alésages (50, 52, 54, 56) sont disposés dans une première et une deuxième ligne au moins, les distances qui séparent un alésage dans la deuxième ligne et les deux alésages les plus proches dans la première ligne, étant approximativement égales.

4. Ensemble selon la revendication 1, dans lequel le bloc de guidage de foret est tourné de 180°entre les première et deuxième orientations.

5. Ensemble selon la revendication 1, dans lequel le rapport de la distance qui sépare les centres d'une paire d'alésages adjacents au moins dans le réseau sur la somme des rayons de ces alésages, n'est pas supérieur à 1 environ.

6. Ensemble selon la revendication 1, dans lequel l'épaisseur du matériau du bloc de guidage (20) entre deux alésages adjacents, n'est pas supérieure à 3 mm environ.

7. Ensemble selon la revendication 1, dans lequel le bloc de guidage (20) présente au moins une ouverture (64, 66) qui s'étend à travers celui-ci et dont la taille transversale est plus petite que celle des alésages de guidage de foret (50, 52, 54, 56), et dans lequel l'ensemble comprend une broche de fixation qui peut être insérée dans l'ouverture et s'étendre dans l'os, de manière à fixer le bloc de guidage sur l'os.

8. Ensemble selon la revendication 1, qui comprend un manchon de guidage de foret (58) amovible qui s'étend à travers l'un des alésages (50, 52, 54, 56) dans le bloc de guidage de foret (20) de manière à protéger le tissu qui entoure l'os d'un foret qui s'étend à travers l'alésage.

9. Ensemble selon la revendication 1, qui comprend :
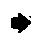 un manchon de guidage (96) qui peut être monté de manière pivotante dans le logement (80), le manchon de guidage présentant un alésage qui s'étend sur sa longueur dans lequel un outil de fraise peut être reçu de telle sorte que la partie tranchante de l'outil de fraise s'étende à partir du manchon vers l'os qui doit être tranché, le manchon étant monté dans le logement pour un mouvement de pivotement autour d'un axe (X) de telle sorte que le déplacement de la partie tranchante de l'outil de fraise soit limité dans un plan qui est perpendiculaire à cet axe ; et
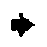 un outil de fraise qui est une adaptation coulissante dans le manchon de guidage.
